# EUROPÄISCHE PATENTSCHRIFT

(11) **EP 0 714 644 B1**
(45) Veröffentlichungstag und Bekanntmachung des Hinweises auf die Patenterteilung: **29.08.2001**
(21) Anmeldenummer: 94810677.8
(22) Anmeldetag: 30.11.1994
(51) Int. Cl.: A61F 2/34

(54) **Innenschale für eine künstliche Hüftgelenkpfanne**
Inner cup for artificial hip acetabulum
Coupule interne d'un joint acétabulaire artificiel

(43) Veröffentlichungstag der Anmeldung: 05.06.1996
(73) Patentinhaber: Sulzer Orthopädie AG, 6340 Baar (CH)
(72) Erfinder: Willi, Roland, CH-8413 Neftenbach (CH)
(74) Vertreter: Sulzer Management AG

(56) Entgegenhaltungen:
- EP-A- 0 445 068
- EP-A- 0 586 335
- EP-A- 0 610 146

## Beschreibung

Die Erfindung betrifft eine Innenschale für eine künstliche Hüftgelenkpfanne gemäss dem Oberbegriff von Anspruch 1. Weiter betrifft die Erfindung eine Hüftgelenkpfanne mit einer erfindungsgemässen Innenschale.

Eine Innenschale gemäß dem Oberbegriff von Anspruch 1 ist aus der EP-A-0 313 762 bekannt.

Künstliche Hüftgelenkpfannen weisen häufig einen zweiteiligen Aufbau auf, einen im Knochen fixierbaren Verankerungskörper beziehungsweise eine Aussenschale und einen Pfannenkörper beziehungsweise eine Innenschale für die Aufnahme eines Gelenkkopfes.

Die aus der EP 0 313 762 A1 bekannte zweiteilige Hüftgelenkpfanne besteht aus einem im Becken mit Hilfe von Knochenschrauben fixierbaren Verankerungskörper in Form einer Halbkugelschale, sowie einem Pfannenkörper, in dessen Pfannenschale ein Gelenkkopf zu liegen kommt. Dabei ist der aus Kunststoff ausgeführte Pfannenkörper durch einen Schnappverschluss im Verankerungskörper fixierbar.
Pfannenkörper aus Kunststoff weisen den Nachteil auf, dass die Pfannenschale durch die langfristige Einwirkung des Gelenkkopfes abgenutzt wird, sodass sich bei einer Reoperation die Notwendigkeit ergeben kann den abgenutzten Pfannenkörper oder die gesamte Hüftgelenkpfanne zu ersetzen.

Der Erfindung liegt die Aufgabe zugrunde, eine Innenschale mit verminderten Abnutzungseigenschaften zu bilden, die sich auch in einen bestehenden Verankerungskörper, der ursprünglich für die Aufnahme einer Innenschale aus Kunststoff konzipiert wurde, einsetzten lässt.

Diese Aufgabe wird erfindungsgemäss durch die kennzeichnenden Merkmale von Anspruch 1 gelöst. Die Unteransprüche 2 bis 4 beziehen sich auf weitere, vorteilhafte Ausbildungen. Die Unteransprüche 5 bis 8 beziehen sich auf eine Hüftgelenkpfanne mit Innenschale.

Die erfindungsgemässe Innenschale besteht aus einem Metall und weist an deren Pol einen gegen aussen vorstehenden Gewindezapfen auf, welcher eine Rotationsachse festlegt und welchen in ein Innengewinde einer Aussenschale einschraubbar ist. Eine solche Innenschale weist den Vorteil auf, dass sich bei einer Reoperation ein aus Kunststoff bestehender Pfannenkörper durch die erfindungsgemässe Innenschale ersetzen lässt, indem die Innenschale in den bestehenden, im Knochen fixierten Verankerungskörper einführbar ist. Der vorstehende Gewindezapfen wird in ein Innengewinde der Aussenschale eingeschraubt, so dass die Innenschale über dieses Verbindungselement in der Aussenschale gehalten ist. Die Innenschale weist weiter einen am Aussenrand angeordneten radial zur Rotationsachse nach aussen verlaufenden Vorsprung auf, welcher Vorsprung mindestens ein Aretierungsteil umfasst, welches bei in die Aussenschale eingesetzter Innenschale direkt oder über ein zusätzliches Zwischenteil auf die Aussenschale wirkt, um ein Drehen der Innenschale um deren Rotationsachse zu verhindern. Damit wird sichergestellt, dass die Innenschale sich nicht von der Aussenschale lösen kann. Zudem ergibt sich im Bereich der Oeffnung von Innenschale und Aussenschale eine gegenseitige mechanische Verbindung, so dass die Innenschale fest in der Aussenschale gehalten ist.

Ein weiterer Vorteil der Erfindung ist darin zu sehen, dass die Innenschale an verschiedenste Ausführungsformen von Aussenschalen befestigbar ist, insbesondere auch an Aussenschalen die auf unterschiedliche Weise mit dem Beckenknochen fixierbar sind. Es können dies Aussenschalen sein, die z.B. mit Knochenschrauben fixierbar sind, oder über ein auf der Aussenfläche angeordnetes Gewinde oder über eine als Pressfit oder Snapfit bezeichnete Befestigungsmethode.

Im folgenden wird die Erfindung anhand eines Ausführungsbeispiels beschrieben. Es zeigen:
- Fig.1a: einen Ausschnitt einer Seitenansicht einer erfindungsgemässen metallischen Innenschale;
- Fig.1b: einen Schnitt durch die erfindungsgemässe Innenschale entlang der Linie A-A gemäss Fig. 1a;
- Fig.2a: einen Ausschnitt einer künstlichen Hüftgelenkpfanne umfassend eine Innenschale und eine Aussenschale;
- Fig.2b: einen Schnitt durch die künstliche Hüftgelenkpfanne entlang der Linie B-B gemäss Fig. 2a;
- Fig.3: eine Seitenansicht einer metallischen Innenschale mit einem Sicherungsring;
- Fig.4: eine Seitenansicht einer Hüftgelenkpfanne mit einem Schnitt durch die Aussenschale;
- Fig.5: eine Detailansicht einer Innenschale mit einem Arvetierungsteil;
- Fig.6: eine Aufsicht auf einen Sicherungsring;

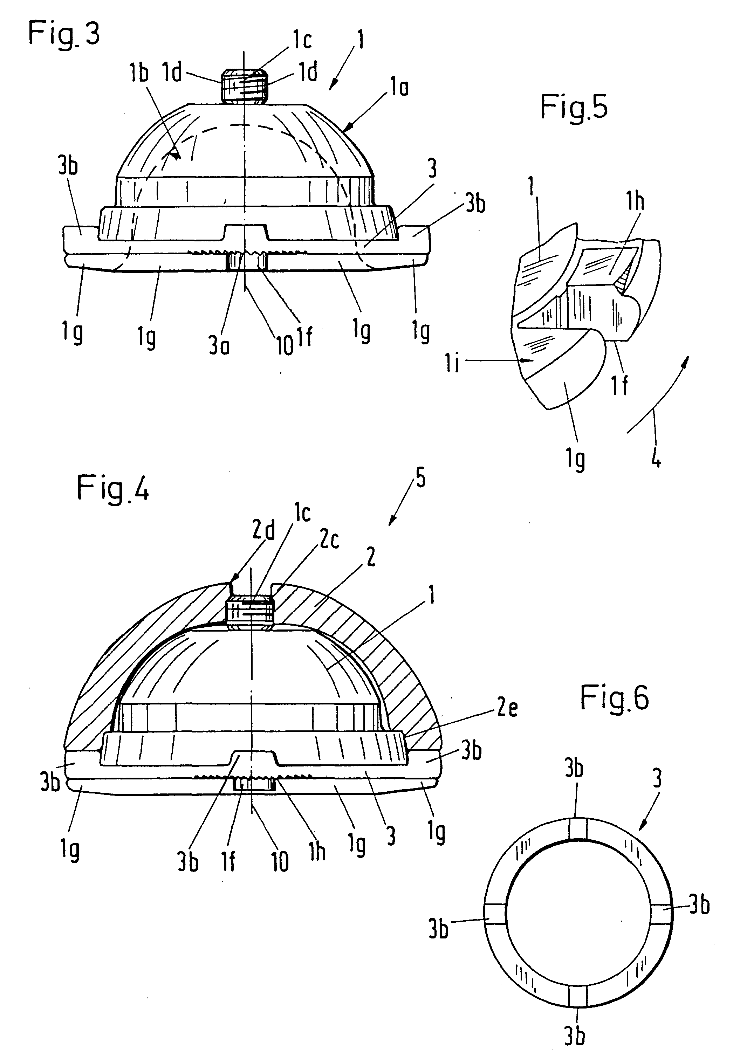

Fig. 4 zeigt eine zusammengesetzte Hüftgelenkpfanne 5, mit einer metallischen Innenschale 1, einem Sicherungsring 3 sowie einer Aussenschale 2, welche zur besseren Darstellung entlang eines Meridianes geschnitten ist. Die Aussenschale 2 weist einen Pol 2c auf, an dem eine zylinderförmige Durchbrechung mit einem Innengewinde 2d angeordnet ist. Weiter weist die Aussenschale 2 eine Oeffnung 2e auf. Die Aussenschale 2 wird möglichst gut im Beckenknochen verankert. Es sind verschiedenste Verankerungsmethoden bekannt, mit der eine Aussenschale 2 im Beckenknochen verankerbar ist, z.B. mittels Knochenschrauben mittels einem Gewinde das auf der Aussenfläche der Aussenschale 2 angeordnet ist, mittels Einzementieren oder durch eine Pressfit oder Snapfit Verbindung.

Eine Innenschale ist in Fig. 3 dargestellt. Die Innenschale 1 weist eine Aussenfläche 1a, sowie einen Innenraum 1b der dazu dient den Gelenkkopf aufzunehmen. Die Innenschale weist an deren Pol ein zylinderförmiges Polteil 1c auf, welches eine Achsrichtung 10 definiert, und welches eine Aussengewinde 1d aufweist. Im Bereich der Oeffnung der Innenschale 1, die üblicherweise im äquatorialem Bereich des Innenraumes 1b angeordnet ist, weist die Innenschale 1 einen Vorsprung 1g auf, der am Aussenrand der Innenschale 1 angeordnet ist und radial zur Rotationsachse 10 nach aussen verläuft. Der Vorsprung 1e kann geschlossen um den Aussenrand der Innenschale 1 verlaufen oder auch Durchbrechungen 1f aufweisen. Der Vorsprung 1g weist auf der dem Gewindezapfen 1c zugewandeten Seite eine kreisringförmige Fläche 1i auf, die teilweise durch Durchbrechungen 1f unterbrochen sein kann. Auf dieser kreisringförmigen Fläche 1i liegt ein Sicherungsring 3 auf, der, wie in Fig. 6 dargestellt ist, kreisförmig ausgeführt ist, und in regelmässigen Abständen Kuppelungsteile 3b aufweist, die in Richtung zum Pol der Innenschale 1 hinweisen. Der Sicherungsring 3 weist auf der dem Pol der Innenschale abgewandeten Seite, wie in Fig. 3 dargestellt, eine sägezahnförmige Oberflächenstruktur 3a auf.

Fig. 5 zeigt in einer Detailansicht der Innenschale 1 einen Vorsprung 1g der Innenschale, mit einer Durchbrechung 1f, sowie der kreisringförmigen Fläche 1i. Auf der kreisringförmigen Fläche 1i ist ein vorstehendes Teil 1h angeordnet, das als ein Arretierungsteil 1h dient. Weiter ist die Drehrichtung 4 der Innenschale beim Einschrauben der Innenschale 1 in die Aussenschale 2 dargestellt, wobei sich bei diesem Vorgang eine Rotation der Innenschale 1 um die Achsrichtung 10 ergibt.

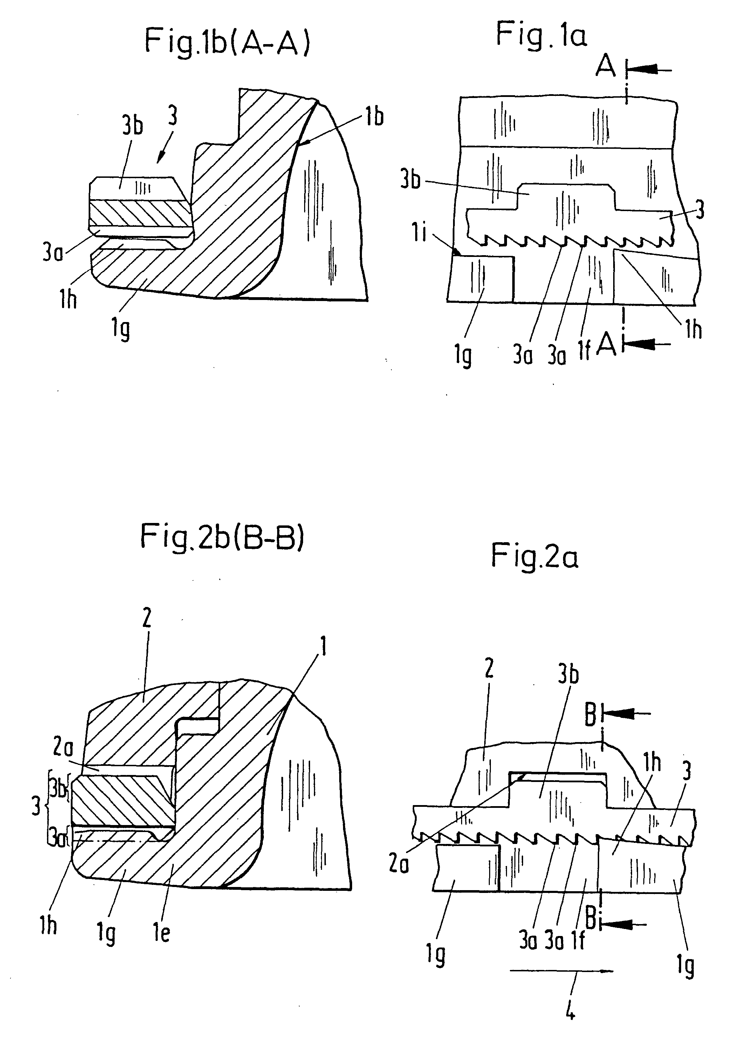

In Fig. 1a ist eine Seiteansicht der Innenschale 1 in einer Detailansicht dargestellt. Die Seitenansicht zeigt den Vorsprung 1g, eine Ausnehmung 1f, die kreisringförmige Fläche li sowie das vorstehende Teil 1h. Weiter ist der Sicherungsring 3 mit Kupplungsteil 3b und sägezahnförmigem Teil 3a dargestellt. In Fig. 1b ist ein Schnitt entlang der Linie A-A gemäss Fig. 1a dargestellt. Von der Innenschale 1 ist der Innenraum 1b ersichtlich, sowie der am Aussenrand angeordnete Vorsprung 1g mit Arretierungsteil 1h. Weiter ist das als Sicherungsring 3 ausgestaltete Zwischenteil dargestellt, mit Kuppelungsteil 3b und dem sägezahnförmigen Teil 3a. Der Verlauf der Zahnung im sägezahnförmigen Teil 3a sowie der Verlauf des vorstehenden Teils 1h sind derart gegenseitig aufeinander angepasst, dass ein gegenseitiges Verdrehen zwischen dem Sicherungsring 3 und der Innenschale 1 verhindert wird, sobald das sägezahnförmige Teil 3a mit dem Arvetierungsteil 1h in Wirkverbindung steht. Im vorliegenden Ausführungsbeispiel ist das Teil 3a sägezahnförmig dargestellt und das Arvetierungsteil 1h als ein vorstehendes Teil. Es gibt natürlich eine Vielzahl von Ausführungsmöglichkeiten dieser beiden Teile um eine gegenseitige Verdrehsicherung zu bewirken, so dass das dargestellte Ausführungsbeispiel nur als eines unter einer Vielzahl von Ausführungsmöglichkeiten zu betrachten ist.

Fig. 2a zeigt einen Ausschnitt aus einer Seitenansicht einer Hüftgelenkpfanne 5 umfassend eine Innenschale 1 mit Vorsprung 1g, Durchbrechung 1f und Arvetierungsteil 1h sowie eine Aussenschale 2 mit Ausnehmung 2a sowie ein Zwischenteil bzw. ein Sicherungsring 3 mit Kuppelungsteil 3b und sägezahnförmigem Teil 3a. In der dargestellten Anordnung ist die Innenschale 1 fest in die Aussenschale 2 verschraubt, wobei das Kupplungsteil 3b in eine Vertiefung bzw. eine Ausnehmung 2a der Aussenschale 2 eingreift, und wobei das sägezahnförmige Teil 3a im Bereich des vorstehenden Teils 1h eine plastische Verformung erfahren hat, so dass die Innenschale 1 und die Aussenschale 2 bezüglich einer gegenseitigen Relativbewegung in Drehrichtung 4 gesichert sind, so dass die Innenschale und die Aussenschale sicher und zuverlässig miteinander verbunden sind. In Fig. 2b ist ein Schnitt entlang der Linie B-B gemäss Fig. 2a dargestellt. Es ist wiederum die Innenschale 1 ersichtlich mit am Aussenrand 1e angeordneten Vorsprung 1g sowie die Aussenschale 2. Weiter ist die Lage des Sicherungsringes 3 ersichtlich, der im oberen Bereich als Kuppelungsteil 3b in die Ausnehmung 2a der Aussenschale 2 eingreift und der im unteren Teil als sägezahnförmiges Teil 3a in Wirkverbindung mit dem Arvetierungsteil bzw. vorstehenden Teil 1h steht. Diese dargestellte Verbindung wirkt als eine Drehsicherung zwischen der Innenschale 1 und der Aussenschale 2, und bewirkt zudem ein Abstützen der Innenschale 1 auf der Aussenschale 2. Das Zwischenteil 3 kann aus Kunststoff oder aus einem Metall gefertigt sein, wobei das Metall einer gewissen Weichheit bzw. Elastizität bedarf um in gewissen Grenzen verformbar zu sein.

Ein weiteres, nicht dargestelltes Ausführungsbeispiel kann derart ausgestaltet sein, dass die Innenschale 1 und die Aussenschale 2 ohne ein Zwischenteil 3 drehsicher miteinander verbunden werden, indem ein entsprechend ausgestaltetes Arvetierungsteil 1h direkt und unmittelbar eine Wirkverbindung zu einer Ausnehmung 2a in der Aussenschale 2 ausübt, und dadurch ein gegenseitiges Verdrehen zwischen Innenschale 1 und Aussenschale 2 verhindert.

Die Innenschale 1 und die Aussenschale 2 werden derart zusammengesetzt, dass der Gewindezapfen 1c der Innenschale in ein Innengewinde 2d der Aussenschale 2 eingeschraubt wird und dass die Innenschale solange eingeschraubt wird, bis sich eine Wirkverbindung zwischen dem Arretierungsteil lh und der Aussenschale 2 ergibt, so dass Innen- und Aussenschale drehfest miteinander verbunden sind. Es kann z.B. auch ein Zwischenteil 3 wie ein ringförmig ausgestattetes Zwischenteil verwenden werden, das derart zwischen Aussenschale 2 und Innenschale 1 angeordnet ist, dass es sich beim zusammenschrauben plastisch verformt, und dadurch ein gegenseitiges Verdrehen von Aussenschale 2 und Innenschale 1 verhindert.

### Stückliste:

- 1: Innenschale 1
- 1a: Aussenfläche der Innenschale
- 1b: Innenfläche der Innenschale
- 1c: zylinderförmiges Polteil der Innenschale
- 1d: Aussengewinde
- 1e: Aussenrand
- 1f: Ausnehmung im Vorspurng der Innenschale
- 1g: Vorsprung der Innenschale
- 1h: vorstehendes Teil
- 2: Aussenschale
- 2c: Pol der Aussenschale
- 2d: Innengewinde
- 2e: äquatorialer Umfang
- 3: Sicherungsring
- 3a: sägezahnförmiges Teil
- 3b: Kuppelungsteil
- 4: Drehrichtung der Innenschale zum Einschrauben
- 5: Hüftgelenkpfanne
- 10: Achsrichtung durch den Pol der Innenschale verlaufend

## Patentansprüche

1. Innenschale (1)mit einem Innenraum (1b) zur Aufnahme eines Gelenkkopfs, welche Innenschale (1) in eine metallische Aussenschale (2) einfügbar ist, um gemeinsam eine Hüftgelenkpfanne (5) zu bilden, wobei die Innenschale an ihrem Pol einen gegen aussen vorstehenden Zapfen aufweist, welcher eine Rotationsachse (10) festlegt, und wobei die Innenschale einen am Aussenrand angeordneten, radial zur Rotationsachse (10) nach aussen verlaufenden Vorsprung (1g) aufweist, der mindestens ein Arretierungsteil (1h) umfasst, welches bei in die Aussenschale (2) eingesetzer Innenschale (1) auf die Aussenschale (2) wirkt, um ein Drehen der Innenschale um deren Rotationsachse zu verhindern
**dadurch gekennzeichnet,**
- dass die Innenschale (1) metallisch ist,
- und dass der am Pol der Innenschale vorgesehene Zapfen als Gewindezapfen (1c) ausgebildet ist, welcher in ein Innengewinde (2d) der Aussenschale (2) einschraubbar ist.

2. Innenschale (1) nach Anspruch 1, **dadurch gekennzeichnet**, dass der Vorsprung (1g) auf der dem Gewindezapfen (1c) zugewandten Seite eine kreisringförmige Fläche (1i) aufweist, und dass das Arretierungsteil (1h) in Richtung zum Gewindezapfen (1c) hin aus der kreisringförmigen Fläche (1i) vorsteht.

3. Innenschale (1) nach Anspruch 2, **dadurch gekennzeichnet**, dass das Arretierungsteil (1h) sägezahnförmig ausgestaltet ist.

4. Innenschale (1) nach einem der Ansprüche 1 bis 3,
**dadurch gekennzeichnet**, dass die kreisringförmige Fläche (1i) Durchbrüche (1f) aufweist, und dass das Arretierungsteil (1h) vorzugsweise anschliessend an einen Durchbruch (1f) angeordnet ist.

5. Hüftgelenkpfanne (5) mit einer Innenschale (1) und einer Aussenschale (2), wobei die Innenschale (1) nach einem der Ansprüche 1 bis 5 ausgebildet ist.

6. Hüftgelenkpfanne (5) nach Anspruch 5, **dadurch gekennzeichnet**, dass sie ein Zwischenteil (3) umfasst, welches als ringförmiges Element ausgestaltet ist, das auf einer kreisringförmigen Fläche (1i) aufliegt, welche auf der dem Gewindezapfen (1c) zugewandten Seite des radial nach aussen Vorsprungs (1g) der Innenschale (1) vorgesehen ist.

7. Hüftgelenkpfanne nach Anspruch 6, **dadurch gekennzeichnet**, dass das Zwischenteil (3) aus einem Metall oder aus einem Kunststoff besteht.

8. Hüftgelenkpfanne nach Anspruch 6 oder 7, **dadurch gekennzeichnet**, dass das Zwischenteil (3) gegen die kreisringförmige Fläche (1i) hin sägezahnförmig ausgestaltet ist.

## Claims

1. Inner shell (1) having an inner space (1b) to receive a joint head, which inner shell (1) is insertable into a metallic outer shell (2) in order to jointly form a hip joint socket (5), with the inner shell (1) having at its pole an outwardly projecting spigot which defines a rotational axis (10) and with the inner shell (1) having a projection (1g) arranged at the outer edge and extending outwardly radially to the rotational axis (10), wherein the projection (1g) includes at least one locking part (1h) which, when the inner shell (1) is fitted into the outer shell (2), acts on the outer shell (2) in order to prevent a rotation of the inner shell (1) about its rotational axis, **characterised in that**
- the inner shell (1) is metallic;
- and in that the spigot provided at the pole of the inner shell is formed as a threaded spigot (1c) which can be screwed into an inner thread (2d) of the outer shell (2).

2. Inner shell (1) in accordance with claim 1, **characterised in that** the projection (1g) has a circular ring-shaped surface (1i) on the surface facing towards the threaded spigot (1c) and in that the locking part (1h) projects out of the surface (1i) in the direction of the threaded spigot (1c).

3. Inner shell (1) in accordance with claim 2, **characterised in that** the locking part (1h) is formed with a sawtooth shape.

4. Inner shell (1) in accordance with any one of the claims 1 to 3,
**characterised in that** the surface (1i) has apertures (1f) and in that the locking part (1h) is preferably arranged adjacent to an aperture (1f).

5. Hip joint socket (5) having an inner shell (1) and an outer shell (2), with the inner shell (1) being formed in accordance with one of the claims 1 to 5.

6. Hip joint socket (5) in accordance with claim 5, **characterised in that** it comprises an intermediate part (3) which is formed as a ring-shaped element lying on a circular surface (1i) provided on the side of the radially outwardly projection (1g) of the inner shell (1) facing the threaded spigot (1c).

7. Hip joint socket in accordance with claim 6, **characterised in that** the intermediate piece (3) is made of a metal or plastic.

8. Hip joint socket in accordance with claim 6 or claim 7,
**characterised in that** the intermediate part (3) is designed with a sawtooth shape towards the circular ring-shaped surface (1i) .

## Revendications

1. Cupule interne (1) avec un espace intérieur (1b) pour la réception d'une tête d'articulation, ladite cupule interne (1) pouvant être insérée dans une cupule externe métallique (2) pour former conjointement une coque acétabulaire (5) où la cupule interne présente à son pôle une goupille faisant saillie vers l'extérieur qui définit un axe de rotation (10) et où la cupule interne présente une saillie (1g) disposée au bord extérieur, s'étendant radialement à l'axe de rotation (10) vers l'extérieur, qui comprend au moins une partie d'arrêt (1h) qui, lorsque la cupule interne (1) est placée dans la cupule externe (2), agit sur la cupule externe (2) pour empêcher une rotation de la cupule interne autour de son axe de rotation, **caractérisée en ce**
- que la cupule interne (1) est métallique,
- et que la goupille prévue au pôle de la cupule interne est réalisée comme goupille filetée (1c) qui peut être vissée dans un filetage interne (2a) de la cupule externe (2).

2. Cupule interne (1) selon la revendication 1, **caractérisée en ce que** la saillie (1g) présente sur le côté orienté vers la goupille filetée (1c) une face circulaire (1i), et en ce que la partie d'arrêt (1h) fait saillie en direction de la goupille filetée (1c) depuis la face circulaire (1i).

3. Cupule interne (1) selon la revendication 2, **caractérisée en ce que** la partie d'arrêt(1h) est réalisée en forme de dents de scie.

4. Cupule interne (1) selon l'une des revendications 1 à 3, **caractérisée en ce que** la face circulaire (1i) présente des perçages (1f), et en ce que la partie d'arrêt (1h) est disposée de préférence à la suite d'un perçage (1f).

5. Coque acétabulaire (5) avec une cupule interne
(1) et une cupule externe (2), où la cupule interne (1) est réalisée selon l'une des revendications 1 à 5.

6. Coque acétabulaire (5) selon la revendication 5, **caractérisée en ce qu**'elle comprend une partie intermédiaire (3) qui est réalisée comme élément annulaire qui repose sur une face circulaire (1i) qui est prévue sur le côté orienté vers la goupille filetée (1c) de la saillie (1g) radialement vers l'extérieur de la cupule interne (1).

7. Coque acétabulaire selon la revendication 6, **caractérisé en ce que** la partie intermédiaire (3) est réalisée en un métal ou en une matière synthétique.

8. Coque acétabulaire selon la revendication 6 ou 7, **caractérisée en ce que** la partie intermédiaire (3) est réalisée en forme de dents de scie vers la face circulaire (1i).
